Europäisches Patentamt

European Patent Office

Office européen des brevets

(19)

(11) **EP 1 284 994 B1**

(12) **FASCICULE DE BREVET EUROPEEN**

(45) Date de publication et mention
de la délivrance du brevet:
**28.07.2004 Bulletin 2004/31**

(51) Int Cl.[7]: **C07K 14/435**, A61K 38/42,
C07K 14/805

(21) Numéro de dépôt: **01936553.5**

(22) Date de dépôt: **17.05.2001**

(86) Numéro de dépôt international:
**PCT/FR2001/001505**

(87) Numéro de publication internationale:
**WO 2001/092320 (06.12.2001 Gazette 2001/49)**

(54) **UTILISATION COMME SUBSTITUT SANGUIN D'UNE HEMOGLOBINE EXTRACELLULAIRE DE POIDS MOLECULAIRE ELEVE**

VERWENDUNG EINES EXTRAZELLULÄREN HÄMOGLOBINS MIT HÖHEREM MOLEKULARGEWICHT ALS BLUTERSATZ

USE OF A HIGH-MOLECULAR-WEIGHT EXTRACELLULAR HAEMOGLOBIN AS BLOOD SUBSTITUTE

(84) Etats contractants désignés:
**AT BE CH CY DE DK ES FI FR GB GR IE IT LI LU MC NL PT SE TR**

(30) Priorité: **31.05.2000 FR 0007031**

(43) Date de publication de la demande:
**26.02.2003 Bulletin 2003/09**

(73) Titulaire: **CENTRE NATIONAL DE LA RECHERCHE SCIENTIFIQUE (CNRS)**
**75794 Paris Cedex 16 (FR)**

(72) Inventeurs:
• **ZAL, Franck**
  **F-29600 Poujean-Morlais (FR)**
• **TOULMOND, André**
  **F-75012 Paris (FR)**
• **LALLIER, François**
  **F-29250 Saint-Pôl de Léon (FR)**

(74) Mandataire: **Grosset-Fournier, Chantal Catherine**
**Grosset-Fournier & Demachy,**
**54, rue Saint-Lazare**
**75009 Paris (FR)**

(56) Documents cités:
**EP-A- 0 784 983**

• **DATABASE CHEMABS [en ligne] CHEMICAL ABSTRACTS SERVICE, COLUMBUS, OHIO, US; HIRSCH, RHODA ELISON ET AL: "A first evaluation of the natural high molecular weight polymeric Lumbricus terrestris hemoglobin as an oxygen carrier" retrieved from STN Database accession no. 127:272538 CA XP002165189 & ARTIF. CELLS, BLOOD SUBSTITUTES, IMMOBILIZATION BIOTECHNOL. (1997), 25(5) 429-444, 1997,**
• **DATABASE CHEMABS [en ligne] CHEMICAL ABSTRACTS SERVICE, COLUMBUS, OHIO, US; ZAL, FRANCK ET AL: "Quaternary structure of the extracellular hemoglobin of the lugworm Arenicola marina. A multi-angle-laser-light-scattering and electrospray-ionization-mass-spectrometry analysis" retrieved from STN Database accession no. 126:291049 CA XP002165190 & EUR. J. BIOCHEM. (1997), 243(1/2), 85-92, 1997, cité dans la demande**
• **DATABASE CHEMABS [en ligne] CHEMICAL ABSTRACTS SERVICE, COLUMBUS, OHIO, US; WAXMAN, LLOYD: "Hemoglobin of Arenicola cristata" retrieved from STN Database accession no. 76:22118 CA XP002165191 & J. BIOL. CHEM. (1971), 246(23), 7318-27, 1971,**

## Description

L'invention concerne l'utilisation pour la préparation d'un substitut sanguin, d'une hémoglobine extracellulaire de poids moléculaire élevé.

L'invention concerne également de nouveaux substituts sanguins, comprenant une hémoglobine extracellulaire de poids moléculaire élevé.

Le sang est un liquide complexe dont la fonction principale est de transporter l'oxygène et le gaz carbonique, afin d'assurer les processus respiratoires. C'est la molécule d'hémoglobine, que l'on trouve dans les hématies, qui assure cette fonction.

La molécule d'hémoglobine de mammifère est formée par l'assemblage de quatre chaînes polypeptidiques fonctionnelles semblables deux à deux (2 chaînes de globine de type $\alpha$ et 2 chaînes de globine de type $\beta$). Chacune de ces chaînes polypeptidiques possède la même structure tertiaire d'une molécule de myoglobine (11).

L'hème, site actif de l'hémoglobine, est un anneau protoporphyrinique tétrapyrrolique, contenant en son centre un unique atome ferreux. L'atome de fer, fixateur de l'oxygène, contracte 6 liaisons de coordinence : quatre avec les atomes d'azote de la porphyrine, une avec l'histidine proximale F8 et une avec la molécule d'oxygène lors de l'oxygénation de la globine.

On est actuellement confronté aux problèmes d'approvisionnement de sang, dus à la diminution des dons du sang par peur de contamination. Ainsi, la recherche de substituts sanguins s'est accélérée au cours des dernières années. On cherche à concevoir des substituts sanguins artificiels capables d'éliminer les risques de transmission de maladies infectieuses, mais également à s'affranchir des problèmes de compatibilité des groupes sanguins.

Jusqu'à présent, les principales voies de recherche concernent la synthèse de produits chimiques d'une part (23) et la synthèse de produits biologiques d'autre part (24,25).

En ce qui concerne la première voie de recherche, on a utilisé les perfluorocarbones (PFC). Les PFC sont des produits chimiques capables de transporter l'oxygène et ils peuvent dissoudre une grande quantité de gaz, comme l'oxygène et le dioxyde de carbone.

Actuellement, on essaye de produire des émulsions de ces produits qui pourraient être dispersés dans le sang d'une façon plus efficace (29-31).

L'avantage des PFC demeure dans leur capacité oxyphorique directement proportionnelle à la quantité d'oxygène se trouvant dans les poumons. Par ailleurs, en raison de l'absence de membrane à traverser, les PFC peuvent transporter l'oxygène plus rapidement vers les tissus. Toutefois, on ne connaît pas les effets à long terme de la rétention de ces produits dans l'organisme. Lorsque ces produits ont été utilisés pour la première fois comme substitut sanguin dans les années 1960 chez la souris (23,28,32), les effets secondaires ont été très importants. Les PFC n'étaient pas éliminés de la circulation d'une façon satisfaisante et s'accumulaient dans les tissus de l'organisme, ce qui provoquait des oedèmes.

Dans les années 1980, on a testé une nouvelle version de PFC en phase clinique. Mais les problèmes de stockage, de coût financier, d'effets secondaires non négligeables et la faible efficacité de ce composé ont empêché l'extension de sa commercialisation (33,34,35).

Récemment, on a mis au point une nouvelle génération de PFC (PFBO perfluorooctylbromide). Un nouveau produit (29) est en cours de test clinique aux Etats-Unis, mais on a déjà constaté qu'une augmentation de la quantité d'oxygène dans le sang peut engendrer une accumulation d'oxygène dans les tissus, ce qui est dangereux pour l'organisme (formation d'oxygène radicalaire de type superoxyde).

Ainsi, malgré les progrès réalisés au fur et à mesure, les effets secondaires de ces composés sont encore trop importants pour être commercialisés à grande échelle.

En ce qui concerne la deuxième voie de recherche, des chercheurs ont travaillé sur la mise au point de substituts sanguins en modifiant la structure de l'hémoglobine naturelle (24,36). Pour obtenir un substitut sanguin de type hémoglobine modifiée, on utilise des hémoglobines provenant de microorganismes génétiquement modifiés, ou d'origine humaine ou animale, notamment la molécule d'hémoglobine bovine. En effet, l'hémoglobine bovine est légèrement différente de l'hémoglobine humaine sur le plan immunologique, mais elle transporte plus facilement l'oxygène vers les tissus. Néanmoins, le risque de contamination virale ou de type encéphalopathie spongiforme reste toujours important.

Pour être fonctionnelle, l'hémoglobine doit être en contact avec un effecteur allostérique le 2,3-diphosphoglycérate (2,3-DPG), présent uniquement à l'intérieur des globules rouges (38). De plus, sans le 2,3-DPG et d'autres éléments présents dans les globules rouges comme la méthémoglobine réductase, l'hémoglobine subit un processus d'auto-oxydation et perd sa capacité à transporter de l'oxygène ou du dioxyde de carbone.

On peut éliminer ces processus en modifiant la structure de l'hémoglobine, et plus précisément en stabilisant les liaisons faibles de la molécule tétramérique entre les deux dimères $\alpha$ et $\beta$ (39). De nombreuses modifications ont été testées : liaison covalente entre deux chaînes $\alpha$, entre deux chaînes $\beta$ ou encore entre $\alpha$ et $\beta$ (40,41).

On a également tenté de polymériser les molécules tétramériques ou de les conjuguer avec un polymère nommé polyéthylène glycol (PEG) (42). Ces modifications ont pour conséquence de stabiliser la molécule et d'augmenter sa taille, empêchant son élimination par les reins.

On a beaucoup étudié les Annélides pour leur hémoglobine extracellulaire (10,44). Ces molécules d'hémoglobine extracellulaire sont présentes chez les trois classes d'Annélides : Polychètes, Oligochètes et Achètes et même chez les Vestimentifères. Ce sont des biopolymères géants, constitués d'environ 200 chaînes polypeptidiques appartenant à 6 ou 7 types différents que l'on regroupe généralement en deux catégories. La première catégorie, comptant 144 à 192 éléments, regroupe les chaînes polypeptidiques dites "fonctionnelles" portant un site actif et capables de lier réversiblement l'oxygène ; ce sont des chaînes de type globine dont les masses sont comprises entre 15 et 18 kDa et qui sont très similaires aux chaînes de type $\alpha$ et $\beta$ de vertébrés. La deuxième catégorie, comptant 36 à 42 éléments, regroupe les chaînes polypeptidiques dites de "structure" possédant peu ou pas de site actif mais permettant l'assemblage des douzièmes.

Les premières images obtenues sur des hémoglobines extracellulaires d'Arénicole (45,46) ont révélé des éléments hexagonaux. Chaque molécule d'hémoglobine est constituée de deux hexagones superposés (47,48) que l'on a nommés bicouche hexagonale (hexagonal bilayer) et chaque hexagone est lui-même formé par l'assemblage de six éléments en forme de goutte d'eau (49,50), nommés structure globulaire creuse (hollow globular structure) (51-54) ou "douzième". La molécule native est formée de douze de ces sous-unités, d'une masse moléculaire d'environ 250 kDa.

On s'est particulièrement intéressé à Arenicola marina, un Annélide Polychète de l'écosystème intertidal. La structure de son hémoglobine extracellulaire est d'ailleurs déjà connue (60).

Des études ont déjà été faites sur l'utilisation de l'hémoglobine extracellulaire du lombric *(Lumbricus terrestris)* comme substitut sanguin (2). Cependant, cette hémoglobine ne conviendrait pas en raison, d'une part, d'un dérèglement probable de la vasodilatation et/ou de la vasoconstriction des vaisseaux sanguins dû à l'absence de résidus cystéines libres (71) et, d'autre part, cette hémoglobine présente une affinité trop faible vis-à-vis de l'oxygène, c'est-à-dire une $P_{50}$ élevée.

A ce jour, aucun des substituts sanguins dont on dispose ne permet d'éviter les problèmes de contamination et de compatibilité des groupes sanguins, tout en étant dépourvu d'effets secondaires.

L'invention permet de remédier à ces inconvénients.

L'invention a pour objet de proposer de nouveaux substituts sanguins permettant d'éliminer les problèmes de pénurie de dons.

L'invention a également pour objet de proposer de nouveaux substituts sanguins permettant d'éviter les problèmes de transmissions de maladies infectieuses lors de dons du sang.

L'invention a également pour objet de nouveaux substituts sanguins permettant de préserver les organes lors de transplantations.

L'invention a également pour objet de nouveaux substituts sanguins permettant de s'affranchir des problèmes de compatibilité des groupes sanguins, notamment lors de transfusions.

L'invention concerne l'utilisation pour la préparation d'un substitut sanguin, d'une hémoglobine extracellulaire de poids moléculaire d'environ 3 à environ 4 millions de daltons, comportant des chaînes de globines polymérisées, contenant des cystéines libres susceptibles de se lier à des groupes NO et/ou SNO et dont la $P_{50}$ est d'environ 6 à environ 7 mm d'Hg à 37°C.

L'invention concerne également un substitut sanguin, notamment substitut sanguin humain, comprenant une hémoglobine extracellulaire de poids moléculaire d'environ 3 à environ 4 millions de daltons, comportant des chaînes de globines polymérisées, contenant des cystéines libres susceptibles de se lier à des groupes NO et/ou SNO et dont la $P_{50}$ est d'environ 6 à environ 7 mm d'Hg à 37°C.

Par "substitut sanguin", on définit un produit biologique capable de se substituer à l'hémoglobine présente dans les globules rouges (ou hématies) et capable d'assurer ses fonctions de transporteur de gaz (oxygène et dioxyde de carbone). Ce substitut sanguin se doit aussi de délivrer l'oxygène aux tissus et de se charger en $CO_2$ à ce niveau pour libérer ce gaz au niveau des surfaces d'échange (poumons).

Par "hémoglobine extracellulaire", on désigne une hémoglobine non contenue dans les cellules et dissoute dans le sang.

Par "chaînes de globines polymérisées", on définit des associations covalentes de chaînes de globines.

Le nombre de cystéines libres susceptibles de se lier à des groupes NO et/ou SNO peut varier d'environ 120 à environ 150, et notamment d'environ 120 à environ 130.

Un test permettant de déterminer la liaison à des groupes NO est par exemple celui utilisé par Jia et al. (71).

Un test permettant de déterminer la liaison à des groupes SNO est par exemple celui utilisé par Jia et al. (71).

La $P_{50}$ est un paramètre permettant de mesurer l'affinité d'un pigment respiratoire pour l'oxygène, qui correspond à 50% de saturation par l'oxygène des sites de liaison d'un pigment respiratoire.

Elle correspond à l'efficacité qu'a l'oxygène pour se fixer sur l'hème.

La mesure de la $P_{50}$ peut être effectuée selon la technique de l'hemox (1).

Selon un mode de réalisation avantageux, dans le substitut sanguin de l'invention, le coefficient de coopérativité de l'hémoglobine extracellulaire est de 2 à 3 ($n_{50}$).

Le coefficient de coopérativité de l'hémoglobine ($n_{50}$) est défini comme étant le paramètre permettant d'estimer la capacité de liaison de l'oxygène par les différents sites actifs des chaînes de globine.

La mesure de $n_{50}$ peut être effectuée sur les courbes de saturation d'un pigment respiratoire par l'oxygène, obtenues à partir de la technique de l'hemox.

Selon un mode de réalisation avantageux, dans le substitut sanguin de l'invention, les chaînes de globine de l'hémoglobine extracellulaire sont stabilisées entre elles, par des liaisons covalentes, notamment des ponts disulfures intermoléculaires, et les chaînes de globine sont auto-stabilisées par des ponts disulfures intramoléculaires.

L'expression "les chaînes de globine de l'hémoglobine extracellulaire sont stabilisées entre elles, par des liaisons covalentes" désigne la présence de liaisons disulfures interchaînes entre deux ou plusieurs chaînes de globine.

L'expression "les chaînes de globine sont auto-stabilisées" désigne la présence de liaisons disulfures intrachaînes sur chaque chaîne de globine.

Selon un mode de réalisation avantageux, dans le substitut sanguin de l'invention, l'hémoglobine extracellulaire comporte des chaînes de structure qui confèrent à l'hémoglobine une structure hexagonale.

Les "chaînes de structure" désignent des chaînes polypeptidiques possédant peu ou pas d'hème, et qui assurent le maintien de la structure hexagonale de la molécule.

Selon un mode de réalisation avantageux, dans le substitut sanguin de l'invention, l'hémoglobine extracellulaire est susceptible de neutraliser des composés toxiques, tel que l'hydrogène sulfuré.

L'expression "l'hémoglobine extracellulaire est susceptible de neutraliser des composés toxiques" désigne la fixation d'hydrogène sulfuré sur des résidus cystéines libres permettant de diminuer, voire éliminer, ce composé du milieu intérieur d'un organisme. Une fois fixé, l'hydrogène sulfuré devient non toxique.

Par "composés toxiques", on définit par exemple un élément chimique ou biologique qui va engendrer des dérèglements physiologiques ou des troubles pathologiques chez un organisme.

Un test permettant de vérifier la neutralisation de composés toxiques, est par exemple celui utilisé dans les deux publications (59,74), test qui fait intervenir un dosage par chromatographie en phase gazeuse.

Selon un mode de réalisation avantageux, dans le substitut sanguin de l'invention, l'hémoglobine extracellulaire ne nécessite pas de cofacteur pour libérer l'oxygène éventuellement fixé sur l'hémoglobine.

L'expression "l'hémoglobine extracellulaire ne nécessite pas de cofacteur" désigne une hémoglobine dissoute dans le sang qui est capable de libérer son oxygène sans l'intervention d'une autre molécule comme c'est le cas pour les hémoglobines intracellulaires qui font, par exemple, intervenir le 2,3-DPG.

L'hémoglobine de vertébrés est contenue dans des cellules anucléées ou globules rouges. A l'intérieur de ces cellules, on trouve comme principal cofacteur le 2,3-DPG qui permet de libérer l'oxygène fixé.

Si le 2,3-DPG se trouvait en présence d'hémoglobine extracellulaire, celui-ci n'aurait aucun effet sur la libération d'oxygène par ce pigment.

Selon un mode de réalisation avantageux, dans le substitut sanguin de l'invention, l'hémoglobine extracellulaire possède les propriétés suivantes :

- elle est non toxique
- elle est dépourvue d'agent pathogène
- elle se conserve à 4°C pendant au moins 6 semaines sans oxydation
- elle est transfusable à tous les types sanguins
- elle est dotée d'un temps de résidence suffisamment long pour assurer la régénération en hémoglobine naturelle de l'organisme dans lequel elle est transfusée
- elle est éliminée par l'organisme dans lequel elle est transfusée sans effet secondaire.

L'expression "non toxique" signifie que le substitut sanguin ne provoque aucun trouble pathologique de type réactions immunitaires, allergiques ou néphrotoxiques.

L'expression "dépourvue d'agent pathogène" désigne l'absence de microorganismes ou de virus identifiés.

L'absence de troubles pathologiques implique indirectement l'absence de pathogènes.

L'expression "se conserve à 4°C pendant au moins 6 semaines sans oxydation" signifie que le site actif et notamment le fer présent au niveau de l'hème et qui intervient dans la liaison de l'oxygène reste sous forme $Fe^{2+}$ (état fonctionnel). L'oxydation du site actif est dû au passage $Fe^{2+} \rightarrow Fe^{3+}$ impliquant une impossibilité de lier l'oxygène.

L'expression "transfusable à tous les types sanguins" désigne l'absence de typage sanguin (système ABO ou rhésus). Cette hémoglobine pourrait être considérée comme une hémoglobine de type donneur universel.

L'expression "dotée d'un temps de résidence suffisamment long pour assurer la régénération en hémoglobine naturelle de l'organisme dans lequel elle est transfusée" désigne la présence de cette hémoglobine dans le système sanguin après au moins 48 heures précédant la transfusion. Ce temps est suffisamment long pour qu'un organisme puisse resynthétiser ses propres globules rouges.

A titre d'illustration, dans le cadre de la transfusion d'un être humain, le temps doit être avantageusement de l'ordre de 48 heures.

L'expression "éliminée par l'organisme dans lequel elle est transfusée sans effet secondaire" signifie que cette hémoglobine extracellulaire semble être éliminée par une voie naturelle n'engendrant aucun trouble pathologique particulier.

Chez les vertébrés, la durée de vie d'un globule rouge est d'environ 120 jours. Le globule rouge est ensuite phagocyté (hémolyse physiologique). L'hémoglobine se transforme alors en biliverdine et en bilirubine qui sont éliminés par la bile.

Aucun des effets secondaires susceptibles d'être rencontrés avec les produits de l'art antérieur, notamment les oedèmes, les problèmes immunogènes et la néphrotoxicité, n'existe dans le cadre de la présente invention.

Selon un mode de réalisation avantageux, dans le substitut sanguin de l'invention, l'hémoglobine extracellulaire provient d'Annélides.

La classification à laquelle on se réfère en utilisant le terme Annélides est celle décrite dans Meglitsch P.A. (1972) (75).

Selon un mode de réalisation avantageux, dans le substitut sanguin de l'invention, l'hémoglobine extracellulaire provient de Arenicola marina.

Dans l'hémoglobine extracellulaire de Arenicola marina, le nombre de cystéines libres susceptibles de se lier à des groupes NO et/ou SNO est égal à 124.

Par ailleurs, il y a, au total, 156 ponts disulfures intra-chaînes sur les chaînes de globine, puisqu'il existe une liaison intra-chaîne (liaison disulfure) sur chaque chaîne de globine et que la molécule est composée de 156 chaînes de type globine (60).

S'agissant de liaisons intermoléculaires, chaque douzième de la molécule est constituée de douze chaînes de type globine associées de la façon suivante : 3 trimères covalents et 3 monomères. Il existe donc 52 liaisons intermoléculaires entre les chaînes de globines.


## DESCRIPTION DES FIGURES


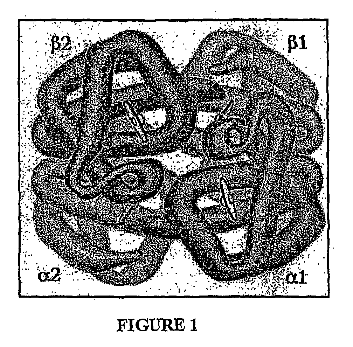

La figure 1 représente la structure de la molécule d'hémoglobine

La molécule d'hémoglobine de mammifère est formée par l'assemblage de quatre chaînes polypeptidiques fonctionnelles semblables deux à deux (deux chaînes de type $\alpha$ et deux chaînes de type $\beta$), ayant chacune la structure tertiaire d'une molécule de myoglobine (11).

Les figures 2A et 2B représentent le modèle de l'hémoglobine bicouche hexagonale (hexagonal bilayer HBL) de Arenicola marina.

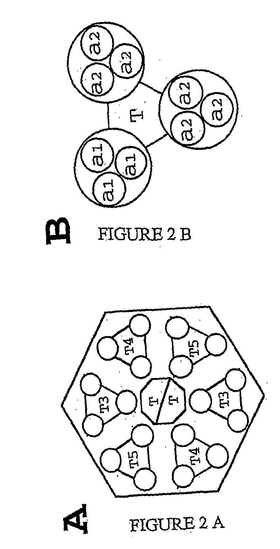

Fig 2A : Vue de face
Tn correspond aux différents trimères composés des chaînes b, c et d de type globine
Fig 2B : détail d'un douzième

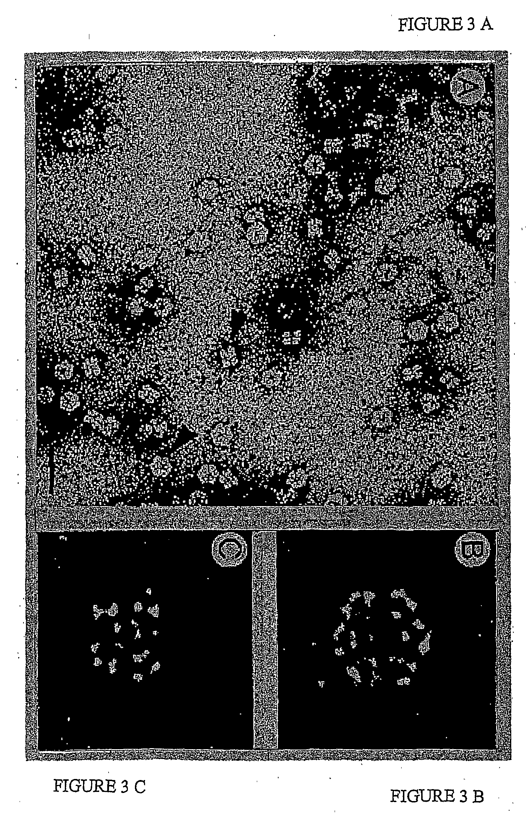

Les figures 3A, 3B et 3C représentent l'hémoglobine de Arenicola marina en microscopie électronique à transmission.

Fig 3A : Vue d'ensemble d'une solution contenant de l'hémoglobine extracellulaire de Arenicola marina.
Fig 3B : Vue de face de la molécule
Fig 3C : Vue de profil

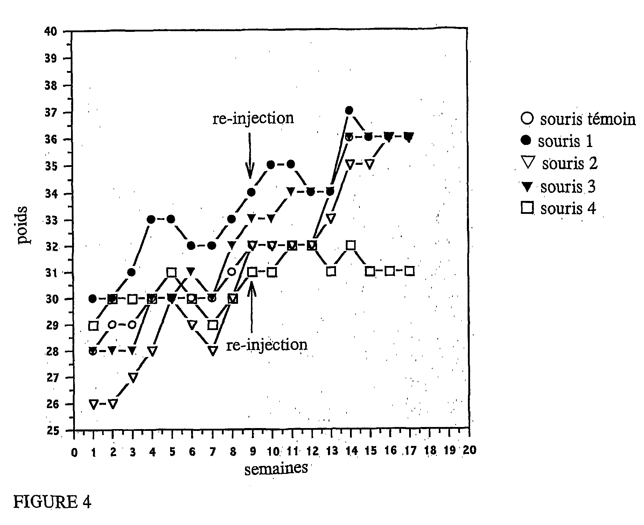

Figure 4 : Surveillance du poids pendant 17 semaines d'un ensemble de 5 souris transfusées avec 1-2g/% d'hémoglobine d'*Arenicola*, comme décrit dans les exemples ci-après.

L'axe des abscisses correspond aux semaines et l'axe des ordonnées correspond au poids. La courbe avec des ronds blancs correspond à la souris témoin, celle avec des ronds noirs à la souris n°1, celle avec des triangles blancs à la souris n°2, celle avec des triangles noirs à la souris n°3, celle avec des carrés blancs à la souris n°4.

Même après l'échange sanguin les souris continuent à croître, la souris contrôle étant le témoin de la bonne forme des animaux.

Après 9 semaines, deux souris sont retransfusées avec les hémoglobines d'*Arenicola marina*. Encore une fois, aucun trouble n'est observé, attestant d'aucune immunoréactivité ou réponse allergique.

## EXEMPLES

### Prélèvements des échantillons d'hémoglobines

Les Arénicoles ont été récoltés à marée basse sur l'estran près de Saint-Pol de Léon, Nord-Finistère, France. Le sang est prélevé au niveau du vaisseau ventral après dissection sur un lit de glace. Les prélèvements sont effectués à l'aide d'une micropipette de verre, reliée à un système d'aspiration à bouche, mis au point par Toulmond (1975) ou de seringues hypodermiques de 1 ml munies d'une aiguille 25Gx5/8". Les échantillons sont collectés sur de la glace. Après une centrifugation à froid (15 000 g pendant 15 min à 4°C) pour éliminer les éventuels débris tissulaires, les surnageants sont soit congelés à -20°C ou dans l'azote liquide, soit immédiatement purifiés.

### Purification des hémoglobines

Avant purification, l'échantillon décongelé est centrifugé pendant 5 min à 5 000 g à 4°C. Après centrifugation, un petit culot est généralement présent, celui-ci est éliminé.

La filtration basse pression (FPLC, Pharmacia, LKB Biotechnology Inc.) d'aliquots de 100 µl du surnageant est réalisée sur une colonne Superose 6-C (Pharmacia, gamme de séparation comprise entre $5.10^3$ et $5.10^6$ Da) ou par simple chromatographie sur une colonne Sephacryl S-500 HR de 2,5 x 100 cm (Amersham Pharmacia Biotech, gamme de séparation comprise entre 40 et 20 000 kDa). Les échantillons sont élués avec le tampon salé *Riftia* développé par Arp et al. (1987) et Fisher et al. (1988). La composition de ce tampon modifié est la suivante, pour un litre: 23,38 g NaCl (400 mM) ; 0,22 g KCl (2,95 mM) ; 7,88 g $MgSO_4$, $7H_2O$ (31,97 mM) ; 1,62 g $CaCl_2$, $2H_2O$ (11,02 mM) et HEPES (50 mM). Le pH est ajusté à pH = 7,0 en ajoutant de l'HCl. Le débit que l'on utilise est généralement de 0,4 à 0,5 ml/min. L'absorbance de l'éluat est suivie à deux longueurs d'ondes : 280 nm (pic d'absorbance des protéines) et 414 nm (pic d'absorbance de l'hémoglobine). Les fractions contenant de l'hème sont concentrées à l'aide de tubes Centricon-100 (15 ml) ou à l'aide d'une cellule à agitation retenant les molécules d'un poids supérieur ou égal à 10 000 Da. Deux purifications suivant le même protocole sont nécessaires pour obtenir des fractions pures.

### Transfusion d'ArHb à des souris

Le but de cette manipulation était d'étudier la possibilité d'utiliser l'hémoglobine extracellulaire de Arenicola marina (ArHb) comme substitut sanguin chez un modèle de vertébrés.

Pour cela, on a utilisé 30 souris mâles C57 BL/6J, adultes reproducteurs, et dont la masse était comprise entre 25 et 40 g. Quatre souris ont servi de contrôle. En général, le volume sanguin d'une souris de ce type est compris entre 1,5 et 2 ml.

Tout d'abord, on a anesthésié les souris au chloroforme après les avoir pesées et clairement identifiées.

On a prélevé ensuite de 200 à 800 µl de sang dans le plexus retro-orbital et le sang de chaque souris a été centrifugé à faible vitesse pour récupérer le plasma (surnageant). Celui-ci a été précieusement conservé pour être réinjecté par la suite à la même souris avec de l'ArHb. L'ArHb préalablement purifiée est dissoute dans le plasma à une concentration de 1,5g%.

Puis, on a injecté dans la veine caudale le mélange ainsi préparé. Sur les souris témoins, après prélèvement d'un volume sanguin, on a injecté le même volume prélevé d'une solution saline isotonique contenant leur plasma respectif.

Enfin, sur 5 souris, on a conservé 10 µl de sang de la souris avant transfusion et 10 µl de sang après transfusion pour l'étude des propriétés fonctionnelles. Sur cinq autres souris, on a fait un prélèvement de sang de 30 à 40 µl dans le plexus orbital après 2 et 48 heures pour l'analyse des propriétés fonctionnelles et la réalisation d'études spectrophotométriques permettant l'identification éventuelle de méthémoglobine.

On a surveillé ces souris pendant trois mois en observant plus particulièrement leur comportement général et leur prise de poids.

On a constaté que les souris transfusées avec de l'ArHb ne mourraient pas et qu'elles avaient un comportement similaire aux souris témoins.

L'analyse des prélèvements sanguins a montré les éléments suivants: i) l'ArHb était toujours présent après 48 heures précédant la transfusion ; ii) aucune modification des propriétés fonctionnelles du sang des souris transfusées ; iii) aucun signe de la présence de méthémoglobine.

### Immunoréactivité

Deux mois après leur première transfusion avec de l'ArHb, une nouvelle injection a été réalisée dans le système vasculaire (2 souris) et d'une façon intrapéritonéale (2 souris) Ces injections de 800 µl contenaient une solution saline isotonique dans laquelle de l'ArHb était dissoute (1-2 g/%).

Aucun trouble n'a pu être observé après leurs sorties d'anesthésies et, à ce jour, ces animaux sont toujours en vie.

Cette absence de réponse immunitaire peut-être liée soit à la taille de cette protéine qui ne permettrait pas l'activation du système immunitaire soit au fait qu'après quelques jours les macrophages ont totalement éliminé ces protéines étrangères.

**Propriétés fonctionnelles**

$P_{50}$

La mesure de la $P_{50}$ a été effectuée selon la technique de l'hemox (1).

$n_{50}$

La mesure de $n_{50}$ a été effectuée sur les courbes de saturation par l'oxygène d'un pigment respiratoire, obtenues à partir de la technique de l'hemox.

Le tableau suivant présente les mesures de $P_{50}$ (affinité) et de $n_{50}$ (coopérativité) pour Arenicola marina en comparaison avec les valeurs de l'hémoglobine humaine correspondante. Ces mesures ont été obtenues in vitro dans les mêmes conditions, pour l'hémoglobine humaine et celle de Arenicola marina.

|  | $P_{50}$ (mm Hg) | $n_{50}$ |
|---|---|---|
| Hémoglobine de Arenicola marina | 6,4 | 2,7 |
| Hémoglobine humaine | 6,1 | 2,6 |

En ce qui concerne Arenicola marina, la valeur indiquée est une moyenne sur trois mesures.

Ces résultats montrent que l'hémoglobine de Arenicola marina et l'hémoglobine humaine (HbA) possèdent des propriétés fonctionnelles similaires sans aucune modification préalable.

**Hémoglobines extracellulaires face au NO/SNO**

**Le Monoxyde d'azote (NO)**

Schématiquement, un vaisseau sanguin peut être représenté par un cylindre constitué de tissus musculaires lisses à l'extérieur, puis d'une couche de cellules endothéliales en contact avec le sang. Cette couche de cellules endothéliales joue un rôle important car elle intervient dans les processus de libération de NO. Le NO est le facteur majeur contrôlant le tonus vasculaire. En diminuant la concentration de NO dans le sang, les vaisseaux seront en état de vasoconstriction, et à l'inverse, une augmentation de NO produira une vasodilatation des vaisseaux (68). Le monoxyde d'azote est également connu comme neuromédiateur (69). Il intervient aussi dans d'autres mécanismes de régulation du métabolisme (70). Les jonctions entres les cellules endothéliales permettent à une hémoglobine tétramérique de traverser cette couche cellulaire et d'être éliminée de la circulation. Par conséquent, comme l'hémoglobine est capable de fixer le monoxyde d'azote, celle-ci, en quittant les vaisseaux, agit comme un puits pour le NO, ce qui engendre des phénomènes de vasoconstriction des vaisseaux mais aussi de nombreux problèmes neurologiques. Actuellement, toutes les solutions d'hémoglobines modifiées (pontées, polymérisées ou encore conjuguées) contiennent une faible proportion d'hémoglobines tétramériques normales traversant la couche de cellules endothéliales. Ce problème est résolu en utilisant des hémoglobines extracellulaires de hauts poids moléculaires comme celles de Arenicola marina qui sont naturellement polymérisées et de taille trop importante pour traverser la paroi des vaisseaux.

**Les groupements thionitrosils (SNO)**

L'hémoglobine des vertébrés possède en plus de son rôle de transporteur d'oxygène, un rôle important dans le transport du NO et du SNO (71). Sommairement, il a été montré que l'oxyhémoglobine avait une plus grande affinité pour le SNO que la déoxyhémoglobine, que la déoxyhémoglobine avait une plus grande affinité pour NO que l'oxyhémoglobine et que le SNO était notamment produit au niveau des poumons et qu'il avait un rôle majeur dans le contrôle de la vasoconstriction et vasodilatation des vaisseaux. Il est intéressant de faire remarquer ici, qu'en ce qui concerne l'hémoglobine extracellulaire de Arenicola marina, on a pu montrer que seules les hémoglobines appartenant à des vers marins colonisant des milieux riches en hydrogène sulfuré avaient les sites (présence de cystéines libres sur des chaînes de type globine) nécessaires pour assurer cette fonction (58). Cette propriété a été étudiée selon la technique de Jia et al. (71).

**Hémoglobines extracellulaires et activité SOD**

Les globules rouges contiennent de nombreuses enzymes telles que des catalases et des superoxydes dismutases (SOD) qui ont un rôle indispensable dans l'inactivation de l'oxygène radicalaire, composé hautement toxique. Cependant, les substituts sanguins actuels ne possèdent pas ces activités puisqu'ils sont localisés hors des hématies. Un déficit d'oxygénation de l'organisme, provoqué par un choc hémorragique ou une ischémie, stimule la production d'hypoxanthine et active la xanthine oxydase. Si cet organisme est alors sous oxygène, la xanthine oxydase transformera l'hypoxanthine en superoxyde qui donnera de l'oxygène radicalaire. L'enzyme superoxyde dismutase aura alors pour rôle de transformer l'oxygène radicalaire en peroxyde d'hydrogène lui même transformé en eau par la catalase. Les premières générations de substituts sanguins étaient dépourvues de ces enzymes provoquant de nombreux effets secondaires. Bien que les nouvelles générations de produits tentent de pallier à ces problèmes, ceux-ci ne sont pas résolus ce qui donne encore un avantage à l'utilisation d'hémoglobines extracellulaires de Arenicola marina. En effet, ces molécules possèdent une activité SOD intrinsèque pouvant être liée à la présence de chaînes de structure (72,73).
On a mesuré l'activité SOD (Superoxyde dismutase) de l'ArHb et on a trouvé des valeurs de l'ordre de 10 U/ mg de protéine.
L'activité SOD a été étudiée par luminescence. Ce dosage se base sur la compétition entre la SOD et une imidazolopyrazine pour l'anion superoxyde. Cet anion, généré par l'action de la xanthine oxydase sur l'hypoxanthine en présence_d'oxygène, peut réagir avec l'imidazolopyrazine et produire de la lumière. En présence de SOD, une partie des anions superoxydes est consommée et l'autre oxyde l'imidazolopyrazine, en excès dans le milieu réactionnel, libérant la lumière mesurée. La luminescence mesurée est donc d'autant élevée que le contenu en SOD de l'échantillon est faible.

$$HPX \text{ (hypoxanthine)} + XOD \text{ (xanthine oxydase)} + O_2 \rightarrow \text{acide urique} + O_2^- \text{ (ion superoxyde)}$$

$$2\, O_2^- + SOD \rightarrow H_2O_2 + O_2$$

$$CL_9 \text{ (coelanterazine)} + O_2^- \rightarrow CL_9 \text{ oxydé} + h\nu$$

Transfusion d'hémoglobine d'*Arenicola* chez les souris

Environ 50% du volume du sang est soutiré et remplacé par 1-2g/% d'hémoglobine d'*Arenicola marina*. Les hémoglobines d'annélides sont dissoutes dans le plasma des animaux ou dans un tampon avant injection. Le volume du substitut injecté est essentiellement le même que le volume initial prélevé de la souris. L'observation la plus surprenante est qu'il n'y a pas d'effets comportementaux ou d'effets physiopathologiques chez ces souris partiellement transfusées avec l'hémoglobine d'*Arenicola marina* (n=30), même 14 mois plus tard (figure 4).

Immunoréactivité

Les souris re-transfusées 9 semaines après la transfusion initiale avec de l'hémoglobine d'*Arenicola marina* ne montrent aucune réponse allergique et aucun décès n'est à déplorer. Dans cet ensemble d'expériences, 200 µg d'hémoglobine sont transfusés par la veine caudale dans 2 souris expérimentales. Après récupération de l'anesthésie, ces souris se comportent normalement. Deux semaines après cette transfusion (c'est-à-dire 12 semaines après la transfusion initiale), les souris sont à nouveau retransfusées avec une solution d'hémoglobines *Arenicola marina* par injection intrapéritonéale, encore une fois aucune allergie ou réponse pathologique n'ont pu être observées (figure 4). On peut donc en conclure que les mécanismes de reconnaissance par les antigènes résultant de la formation d'anticorps ne sont pas activés par une protéine de cette taille ou que les macrophages ont éliminé sans problème apparent cette protéine de grande taille.

Ces nouveaux résultats conduisent à la conclusion que la taille de ces molécules peut être un facteur déterminant pour que l'hémoglobine de *Arenicola marina*, puisse fonctionner de manière non-toxique chez les vertébrés.

<u>**REFERENCES**</u>

1. A. Toulmond et al., Biol. Bull. <u>179</u> 366-373 (1990)

2. R.E. Hirsch, L.A. Jelicks, B.A. Wittenberg, D.K. Kaul, H.L. Shear and J.P. Harrington, Artificial Cells, Blood Substitutes & Immobilization Biotechnology <u>25</u> 429-444 (1997).

10. N.B. Terwilliger, <u>Molecular Structure of the extracellular heme proteins.</u>, Vol. 13, C.P. Mangum (Ed), 193-229, Springer-Verlag, Berlin (1992).

11. R.E. Dickerson and I. Geis, <u>Hemoglobin: Structure, function, evolution, and pathology.</u>, Benjamin/Cummings, Menlo Park, CA (1983).

23. L.C.J. Clark and F. Gollan, Science <u>152</u> 1755 (1966).

24. T.M.S. Chang, <u>Hemoglobin Corpuscles</u>, McGill University. (1957).

25. T.M.S. Chang, Science <u>146</u> 524-525 (1964).

28. R.P. Geyer, R.G. Monroe and K. Taylor, <u>Survival of rats totally perfused with a fluorocarbon-detergent prepa-ration.</u>, J.C. Norman, J. Folkman, W.G. Hardisson, L.E. Rudolf and F.J. Veith (Eds), 85-96, Appleton Century Crofts, New York (1968).

29. J.G. Reiss, Vox Sang <u>61</u> 225-239 (1991).

30. J.G. Reiss, Artificial Cells, Blood substitutes & Immobilization Biotechnology, An International Journal <u>22</u> 945-1511 (1994).

31. T.H. Goodin, E.B. Grossbard, R.J. Kaufman, T.J. Richard, R.J. Kolata, J.S. Allen and T.E. Layton, Crit Care Med <u>22</u> 680-689 (1994).

32. H. Sloviter and T. Kamimoto, Nature <u>216</u> 458 (1967).

33. R. Naito and Y. K., <u>An improved perfluorodecalin emulsion. In Blood Substitutes and Plasma Expanders</u>, G. A. Jamieson and T.J. Greenwalt (Eds), 81, Alan R Liss Inc., New York (1978).

34. T. Mitsuno and R. Naito, <u>Perfluorochemical Blood Substitutes.</u>, Excerpta Medica, Amsterdam (1979).

35. T. Mitsuno and H. Ohyanagi, <u>Present status of clinical studies of fluosol-DA (20%) in Japan,</u> K.K. Tremper (Ed), 169-184, Little Brown & Co, Boston (1985).

36. T.M.S. Chang, <u>Blood substitutes: principales, methods, products and clinical trials,</u> Vol. I, Karger, Basel, Switzerland (1997).

38. R.E. Dickerson and I. Geis, <u>Hemoglobin: structure, function, evolution and pathology.</u>, Benjamin/Cummings, Menlo Park (1983).

39. T.M.S. Chang, Biochem. Biophys. Res. Com. <u>44</u> 1531-1533 (1971).

40. J.W. Payne, Biochem J. <u>135</u> 866-873 (1973).

41. H.F. Bunn and J.H. Jandl, Trans Assoc Am Physicians <u>81</u> 147 (1968).

42. K. Nho, D. Glower, S. Bredehoeft, H. Shankar, R. Shorr and A. Abuchowski, Biomaterials, Artificial Cells and Immobilization Biotechnology, An International Journal <u>20</u> 511-524 (1992).

44. J.N. Lamy, B.N. Green, A. Toulmond, J.S. Wall, R.E. Weber and S.N. Vinogradov, Chem. Rev. <u>96</u> 3113-3124 (1996).

45. J. Roche, M. Bessis and J.P. Thiery, Biochim. Biophys. Acta <u>41</u> 182-184 (1960).

46. J. Roche, M.T. Bessis and J.P. Thiery, C. R. Soc. Biol. <u>154</u> 73-80 (1960).

47. O. Levin, J. Mol. Biol. <u>6</u> 95-101 (1963).

48. J. Roche, <u>Electron microscope studies on high molecular weight erythrocruorins (invertebrate hemoglobins) and chlorocruorins of annelids.</u>, D.A. Munday (Ed), 62-80, Pergamon Press, Oxford (1965).

49. E.F.J. Van Bruggen and R.E. Weber, Biochim. Biophys. Acta <u>359</u> 210-212 (1974).

50. O.H. Kapp and A.V. Crewe, Biochim. Biophys. Acta <u>789</u> 294-301 (1984).

51. F. De Haas, F. Zal, V. You, F.H. Lallier, A. Toulmond and J.N. Lamy, J. Mol. Biol. <u>264</u> 111-120 (1996).

52. F. De Haas, F. Zal, F.H. Lallier, A. Toulmond and J.N. Lamy, Proteins-structure fonction and genetics, <u>3</u> 241-256 (1996).

53. F. De Haas, N. Boisset, J.C. Taveau, O. Lambert, S.N. Vinogradov and J.N. Lamy, Biophys. J. <u>70</u> 1973-1984 (1996).

54. F. De Haas, J.-C. Taveau, N. Boisset, O. Lambert, S.N. Vinogradov and J.N. Lamy, J. Mol. Biol. 255 140-153 (1996).

58. F. Zal, Structure des hémoglobines extracellulaires d'Annélides et de Vestimentifères colonisant des milieux extrêmes. Les hémoglobines face aux sulfures., Thèse de l'Université Pierre-et-Marie-Curie (Paris VI) (1996).

59. F. Zal et al., Proteins: Structure, Function, and Genetics 29 562-574 (1997).

60. F. Zal, B.N. Green, F.H. Lallier, S.N. Vinogradov and A. Toulmond, Eur. J. Biochem. 243 85-92 (1997).

66. O. Ouchterlony and L.A. Nilsson, Immunochemistry, Vol. 10, D.M. Weir, L.A. Herzenberg and C. Blackwell (Eds), 32, Blackwell, Oxford (1986).

68. J.G. Umans and R. Levi, Annu. Rev. Physiol. 57 771-790 (1995).

69. J. Garthwaite and C.L. Boulton, Annu. Rev. Physiol. 57 683-706 (1995).

70. C. Nathan, FASEB J. 6 3051-3064 (1992).

71. L. Jia, C. Bonaventura, J. Bonaventura and J.S. Stamler, Nature 380 221-226 (1996).

72. S.I. Liochev, A.R. Ruchumov, S.N. Vinogradov and I. Fridovich, Archives of Biochemistry and Biophysics 330 281-284 (1996).

73. J. Blum and I. Fridovich, Archives of Biochemistry and Biophysics 228 617-620 (1984).

74. F. Zal et al., Proc. Natl. Acad. Sci, USA 95 8997-9002 (1998).

75. P.A. Meglitsch, Invertebrate Zoology, Oxford University Press, Oxford, 834 p. (1972)

**Revendications**

1. Utilisation pour la préparation d'un substitut sanguin, d'une hémoglobine extracellulaire de poids moléculaire d'environ 3 à environ 4 millions de daltons, comportant des chaînes de globines polymérisées, contenant des cystéines libres susceptibles de se lier à des groupes NO et/ou SNO et dont la $P_{50}$ est d'environ 6 à environ 7 mm d'Hg à 37°C.

2. Substitut sanguin, notamment substitut sanguin humain, comprenant une hémoglobine extracellulaire de poids moléculaire d'environ 3 à environ 4 millions de daltons, comportant des chaînes de globines polymérisées, contenant des cystéines libres susceptibles de se lier à des groupes NO et/ou SNO et dont la $P_{50}$ est d'environ 6 à environ 7 mm d'Hg à 37°C.

3. Substitut sanguin selon la revendication 2, dans lequel le coefficient de coopérativité de l'hémoglobine extracellulaire est de 2 à 3 ($n_{50}$).

4. Substitut sanguin selon la revendication 2 ou 3, dans lequel les chaînes de globine de l'hémoglobine extracellulaire sont stabilisées entre elles, par des liaisons covalentes, notamment des ponts disulfures intermoléculaires, et les chaînes de globine sont auto-stabilisées par des ponts disulfures intramoléculaires.

5. Substitut sanguin selon l'une des revendications 2 à 4, dans lequel l'hémoglobine extracellulaire comporte des chaînes de structure qui confèrent à l'hémoglobine une structure hexagonale.

6. Substitut sanguin selon l'une des revendications 2 à 5, dans lequel l'hémoglobine extracellulaire est susceptible de neutraliser des composés toxiques, tel que l'hydrogène sulfuré.

7. Substitut sanguin selon l'une des revendications 2 à 6, dans lequel l'hémoglobine extracellulaire ne nécessite pas de cofacteur pour libérer l'oxygène éventuellement fixé sur l'hémoglobine.

8. Substitut sanguin selon l'une des revendications 2 à 7, dans lequel l'hémoglobine extracellulaire possède les propriétés suivantes :

   - elle est non toxique
   - elle est dépourvue d'agent pathogène
   - elle se conserve à 4°C pendant au moins 6 semaines sans oxydation
   - elle est transfusable à tous les types sanguins
   - elle est dotée d'un temps de résidence suffisamment long pour assurer la régénération en hémoglobine naturelle de l'organisme dans lequel elle est transfusée
   - elle est éliminée par l'organisme dans lequel elle est transfusée sans effet secondaire

9. Substitut sanguin selon l'une des revendications 2 à 8, dans lequel l'hémoglobine extracellulaire provient d'Annélides.

10. Substitut sanguin selon la revendication 9, dans lequel l'hémoglobine extracellulaire provient d'Arenicola marina.

**Patentansprüche**

1. Verwendung eines extrazellulären Hämoglobins mit einem Molargewicht von ungefähr 3 bis ungefähr 4 Millionen Dalton für die Herstellung eines Blutersatzes, das Ketten polymerisierter Globine aufweist, die freie Cysteine enthalten, die sich an NO- und/oder SNO-Gruppen binden können, und deren $P_{50}$ bei 37°C ungefähr 6 bis ungefähr 7 mm Hg ist.

2. Blutersatz, insbesondere Blutersatz für Menschen mit einem extrazellulären Hämoglobin mit einem Molekulargewicht von ungefähr 3 bis ungefähr 4 Millionen Dalton, das Ketten polymerisierter Globine aufweist, die freie Cysteine enthalten, die sich mit NO- und/oder SNO-Gruppen binden können, und deren $P_{50}$ ungefähr bis ungefähr 7 mm Hg bei 37°C ist.

3. Blutersatz nach Anspruch 2, wobei der Kooperationskoeffizient des extrazellulären Hämoglobins 2 bis 3 ($n_{50}$) ist.

4. Blutersatz nach Anspruch 2 oder 3, bei dem die Globinketten des extrazellulären Hämoglobins miteinander durch kovalente Bindungen, insbesondere intermolekulare Disulfidbrücken stabilisiert sind, und die Globinketten durch intramolekulare Disulfidbrücken autostabilisiert sind.

5. Blutersatz nach einem der Ansprüche 2 bis 4, bei dem das extrazelluläre Hämoglobin Strukturketten besitzt, die dem Hämoglobin eine hexagonale Struktur verleihen.

6. Blutersatz nach einem der Ansprüche 2 bis 5, bei dem das extrazelluläre Hämoglobin geeignet ist, toxische Verbindungen, wie Schwefelwasserstoff, zu neutralisieren.

7. Blutersatz nach einem der Ansprüche 2 bis 6, bei dem das extrazelluläre Hämoglobin keinen Cofaktor zum Freisetzen des Sauerstoffes benötigt, der gegebenenfalls am Hämoglobin fixiert ist.

8. Blutersatz nach einem der Ansprüche 2 bis 7, bei dem das extrazelluläre Hämoglobin die folgenden Eigenschaften besitzt:

   — es ist nicht toxisch,
   — es ist frei von pathogenem Mittel,
   — es hält sich bei 4°C während wenigstens sechs Wochen ohne Oxidation,
   — es ist in alle Arten von Blut transfundierbar,
   — es ist mit einer Verweilzeit dotiert, die ausreichend lang ist, um die Regeneration an natürlichem Hämoglobin des Organismus zu sichern, in das es transfundiert ist,
   — es wird ohne Nebeneffekt durch den Organismus ausgeschieden, in den es transfundiert wurde.

9. Blutersatz nach einem der Ansprüche 2 bis 8, bei dem das extrazelluläre Hämoglobin von Anneliden stammt.

10. Blutersatz nach Anspruch 9, bei dem das extrazelluläre Hämoglobin von *Arenicola marina* stammt.

**Claims**

1. Use for the preparation of a blood substitute, of an extracellular haemoglobin having a molecular weight of approximately 3 to approximately 4 million daltons, comprising chains of polymerised globins, containing free cysteines capable of binding to NO and/or SNO groups, and having a $P_{50}$ of approximately 6 to approximately 7 mm Hg at 37°C.

2. Blood substitute, in particular human blood substitute, comprising an extracellular haemoglobin having a molecular weight of approximately 3 to approximately 4 million daltons, comprising chains of polymerised globins, containing free cysteines capable of binding to NO and/or SNO groups, and having a $P_{50}$ of approximately 6 to approximately 7 mm Hg at 37°C.

3. Blood substitute according to claim 2, wherein the extracellular haemoglobin cooperativity coefficient is 2 to 3 ($n_{50}$).

4. Blood substitute according to claim 2 or 3, wherein the globin chains of extracellular haemoglobin are stabilised between themselves, by covalent bonds, in particular intermolecular disulphide bridges, and the globin chains are auto-stabilised by intramolecular disulphide bridges.

5. Blood substitute according to one of claims 2 to 4, wherein the extracellular haemoglobin comprises structural chains which confer a hexagonal structure on the haemoglobin.

6. Blood substitute according to one of claims 2 to 5, wherein the extracellular haemoglobin is capable of neutralising toxic compounds, such as hydrogen sulphide.

7. Blood substitute according to one of claims 2 to 6, wherein the extracellular haemoglobin does not necessitate any cofactor to release any oxygen possibly fixed onto the haemoglobin.

8. Blood substitute according to one of claims 2 to 7, wherein the extracellular haemoglobin possesses the following properties:

   - it is non-toxic
   - it has no pathogenic agent
   - it keeps for at least 6 weeks at 4°C without oxidation
   - it is transfusable into all blood types
   - it has a sufficiently long residence time to ensure regeneration into natural haemoglobin of the organism into which it is transfused
   - it is eliminated by the organism into which it is transfused without side effects.

9. Blood substitute according to one of claims 2 to 8, wherein the extracellular haemoglobin comes from Annelids.

10. Blood substitute according to claim 9, wherein the extracellular haemoglobin comes from Arenicola marina.

FIGURE 1

FIGURE 2 B

FIGURE 2 A

FIGURE 3 A

FIGURE 3 C

FIGURE 3 B

FIGURE 4